# EUROPEAN PATENT SPECIFICATION

(11) **EP 3 703 800 B1**
(45) Date of publication and mention of the grant of the patent: **21.02.2024**
(21) Application number: 18807561.8
(22) Date of filing: 05.11.2018
(51) Int. Cl.: A61M 16/10

(54) **HEAT AND MOISTURE EXCHANGE MEDIA**
WÄRME- UND FEUCHTIGKEITSAUSTAUSCHMEDIUM
MILIEUX D'ÉCHANGE DE CHALEUR ET D'HUMIDITÉ

(30) Priority: 03.11.2017 GB 201718286
(43) Date of publication of application: 09.09.2020
(73) Proprietor: Intersurgical AG, 9490 Vaduz (LI)
(72) Inventor: MICHNOVIC, Arnold, 18170 Pabrade (LT); GRUNDINSKIENE, Loreta, 18170 Pabrade (LT); BOOTH, Christopher Edgerley, Wokingham, Berkshire RG41 2RZ (GB)
(74) Representative: Adamson Jones
(86) International application number: PCT/EP2018/080211
(87) International publication number: WO 2019/086682

(56) References cited:
- EP-A1- 2 647 401
- EP-A1- 2 705 949
- WO-A2-2010/079380
- US-A1- 2013 068 219

## Description

This invention relates to heat and moisture exchange (HME) materials, and methods for their manufacture, as defined by the appended claims.

During normal breathing, gas enters the body through the mouth and/or nose and is heated and humidified as it passes through the airways and into the lungs. However, when the upper airways are bypassed, such as during tracheotomy or artificial ventilation with a respirator or anaesthesia apparatus, inhaled gas may be inadequately heated and humidified before it reaches the lower airways and lungs. The exposure of the lower airways and lungs to inadequately heated and humidified gas may irritate mucosal surfaces and impair mucosal function, leading to discomfort and increased susceptibility to respiratory tract infection.

Respiratory gas may be passively heated and humidified prior to inhalation using HME devices comprising an HME medium capable of retaining and releasing heat and moisture. Respiratory gas passes through the HME medium such that heat and moisture picked up by exhaled gas in the airways of the patient is transferred to the HME medium, and is then transferred to gas as it passes through the HME medium in the opposite direction during inhalation.

HME media may be formed from layers of sheet material, or from a foam, possibly with a hygroscopic additive to enhance the ability of the HME media to retain moisture.

For the purposes of this application, all references to HME material should be understood to refer to sheet material, most conventionally cellulose based paper containing a fibrous material such as cotton, rather than to foam. HME media are typically formed by stacking multiple layers of HME material adjacent to one another, such as by winding one or more strips of HME material into a coil. The HME medium is then positioned in an HME device such that gas flow is primarily in a transverse direction relative to the plane of the HME material such that gas flows primarily through the gaps between adjacent layers of the HME material in the HME media.

HME materials are typically corrugated to maintain separation between the adjacent layers of HME material and hence reduce the resistance of the HME medium to gas flow and increase the surface area of the HME material that is available for heat and moisture exchange. Such corrugations take the form of continuous/uninterrupted ridges running across the full width of the HME material. Typically, they are provided as straight ridges either perpendicular or at an oblique angle to the axis of the HME material. However, in some cases the ridges forming the corrugations may be in a "V" shaped or zigzag pattern, or may be curved.

HME materials are conventionally corrugated by passing a strip of the material between two meshing gears, although the integrity of corrugations formed by this method can be affected by the relative humidity at the time the corrugations are formed. Accordingly, it is often necessary to carefully monitor and/or control relative humidity during the manufacture of HME media using this method in order to ensure that robust corrugations are produced and the performance of the eventual HME media is consistent. The corrugated HME material may also be bonded to an uncorrugated material with adhesive in order to retain the HME material in its corrugated conformation.

In addition, placing the HME material under tension may result in corrugations becoming flattened and hence losing their ability to separate the adjacent layers of HME material. This is especially problematic for relatively small HME media in the form of a coil, as the HME material must be coiled relatively tightly in order to prevent the formation of an opening at the centre of the HME media.

There has now been devised an improved HME material and method of manufacturing an HME material that overcomes or substantially mitigates the above-mentioned and/or other problems associated with the prior art.

Described is an HME material comprising a plurality of discrete protrusions extending from at least part of one surface of the HME material.

In the context of this application, 'discrete protrusions' are intended to be distinguished from typical corrugations or similar, which have a dimension extending across substantially the entire surface of the material in one direction. Discrete protrusions, in contrast, can be approximated as separate point-like protrusions or protrusions with a closed loop boundary within a surface of the HME material.

The HME material will usually have a length and a width, and a plurality of protrusions may be provided both along the length and across the width of the HME material. The plurality of protrusions may thus be provided in a two-dimensional array on said surface of the HME material. Each protrusion may define a closed loop outer boundary on the surface of the material between opposing edges of the HME material.

The HME material may be for the manufacture of an HME medium for inclusion into an HME device, in particular for use in heat and moisture exchange with respiratory gases. The HME material, HME medium and HME device may be used to heat and humidify respiratory gases, for example during tracheotomy or artificial ventilation with a respirator or anaesthesia apparatus.

The plurality of protrusions extending from at least part of one surface of the HME material are able to maintain separation between the adjacent layers of HME material in an HME medium and hence reduce the resistance of the HME medium to gas flow and increase the surface area of the HME material that is available for heat and moisture exchange.

The integrity of such protrusions in the HME material are less susceptible to the effects of relative humidity at the time of their formation than corrugations. In addition, the protrusions in the HME material have less tendency to flatten than corrugations and hence retain the ability to maintain separation between adjacent layers of HME material even when placed under relatively high tension, such as when it is wound into a tight coil in order to form a relatively small HME medium. The protrusions are preferably formed of the same material as the HME material. In particular, the protrusions are preferably regions of the HME material that have been deformed such that the protrusions are formed of the HME material. The HME material may therefore comprise indentations on the opposite surface to each of the protrusions. Such protrusions may be formed by embossing the HME material. The protrusions according to the invention are completely surrounded by substantially non-protruding HME material.

The regions of the HME material from which each protrusion is formed may be any of a wide variety of shapes, such as circular, elliptical, square, rectangular, rhomboid or any combination of these. The regions of the HME material from which each protrusion is formed may have a maximum or largest dimension (for example a diameter, major diameter, maximum length/width, etc) of at least 0.4 mm, at least 0.6 mm or at least 0.8 mm, and no more than 5.0 mm, no more than 3.0 mm or no more than 2.0 mm. In particular, the regions of the HME material from which each protrusion is formed may have a maximum or largest dimension of between 0.4 mm and 5.0 mm, between 0.6 mm and 3.0 mm or between 0.8 mm and 2.0 mm. In particular, where the regions of the HME material from which each protrusion is formed are circular in shape, the maximum dimension corresponds to the diameter of each protrusion.

According to the invention a single protrusion does not span the entire width of the HME material and, typically, a group of protrusions provided across the width of the HME material similarly do not span the entire width. In any case, the protrusions are preferably arranged such that they are not prone to flattening when the HME material is placed under tension.

The protrusions may be any of a wide variety of shapes, such as hemispherical, frustoconical, cylindrical, pyramidal, cuboid, hemicylindrical or any combination of these.

The maximum distance by which the protrusions protrude from the surface of the HME material is an important factor in their ability to maintain the separation between adjacent layers of HME material. In particular, the protrusions may protrude at least 0.1 mm, at least 0.3 mm or at least 0.5 mm, and preferably no more than 1.0 mm, no more than 0.8 mm and no more than 0.6 mm from the surface of the HME material. Accordingly, the protrusions may protrude from the surface of the HME material by between 0.1 mm and 1.0 mm, between 0.3 mm and 0.8 mm or between 0.5 mm and 0.6 mm.

The tolerance of the materials conventionally used to form HME materials is such that there may be a wide variation in the distance by which the protrusions formed under identical conditions protrude from the surface of the HME material. In particular, the distance by which the protrusions protrude from the surface of the HME material may vary by up to 20%, up to 15%, up to 10% or up to 5%.

Protrusions are preferably distributed across substantially the whole of at least one surface of the HME material. Protrusions may be present on both surfaces of the HME material. The proportion of the area of the HME material on which the protrusions are formed may be least 5%, at least 10% or at least 20%, and may be less than 50%, less than 40% or less than 30%. Accordingly, the proportion of the surface area of the HME material on which the protrusions are formed may be between 5% and 50%, between 10% and 40% or between 20% and 30%.

The protrusions are preferably distributed regularly on the surface of the HME material in order to provide even separation of the adjacent layers of the HME material. A wide variety of distributions may be suitable and in particular the protrusions may be distributed in multiple, such as 3, 4, 5, 6, 7 or 8, rows. The rows may be substantially parallel to each other and may extend along the length of the HME material. The protrusions in each row may be in line with, or offset from, the protrusions in the adjacent rows. The separation between each row may be similar to the separation between the protrusions within each row, such that the protrusions are arranged in a regular grid pattern.

The density of the protrusions on the surface of the HME material may vary significantly depending on the size and shape of the protrusions. However, the density of the protrusions per cm² may be at least 3, at least 5, at least 8 or at least 10, and less than 50, less than 40, less than 30 or less than 20. The density of the projections per cm² on the surface of the HME material may be between 3 and 50, between 5 and 40, between 8 and 30 or between 10 and 20.

The protrusions may further comprise a perforation in the HME material. The perforations may be any of a wide variety of shapes, such as circular, elliptical, square, rectangular, rhomboid or any combination of these. The perforations may have a maximum dimension of at least 0.4 mm, at least 0.6 mm or at least 0.8 mm, and no more than 5.0 mm, no more than 3.0 mm or no more than 2.0 mm. In particular, the perforations may have a maximum dimension of between 0.4 mm and 5.0 mm, between 0.6 mm and 3.0 mm or between 0.8 mm and 2.0 mm. In particular, where the perforations are circular in shape, the maximum dimension corresponds to the diameter of the perforations.

At least a portion of the HME material around the edge of each perforation may protrude from the surface of the HME material. For example, about 25%, about 50% or about 75% of the HME material around the edge of each perforation may protrude. However, substantially the entire edge of each perforation may protrude from the surface of the HME material, such that the protrusions may be substantially in the form of a cone or pyramid with the perforation located at their apex.

The perforations may be in the form of cuts in the HME material. In particular, the perforations may comprise a cut that partially surrounds a region of HME material that thus forms a flap that may extend from the surface of the HME material. For example, the cut may be C-shaped, such that the flap is substantially circular in shape, or V-shaped, such that the flap is substantially triangular in shape.

The formation of perforations in the form of cuts does not involve punching out portions of the HME material and hence tends to produce less swarf and loose fragments than alternative perforation methods.

The HME material may be formed of any material that is capable of retaining and releasing heat and moisture. The HME material is generally a porous material, and in particular is typically a fibrous material, such as paper and particularly cellulose paper. However, the HME material may be formed of, or also include, other natural fibres such as cotton, or synthetic fibres such as polyamine, polyester, polyurethane, polyacrylonitrile and polyvinyl alcohol, or any combination of these.

The HME material may include additives, such as hygroscopic materials that improve the ability of the HME material to retain moisture. In particular, suitable hygroscopic materials include polyols such as glycols and glycerine, hygroscopic polymers such as polyvinylpyrrolidone, polyacrylic acid and polyvinyl alcohol, and hygroscopic salts such as polyacrylate, calcium chloride, potassium chloride and lithium chloride, or any combination of the above.

HME material having greater hygroscopic material content has enhanced ability to retain and release moisture, and hence has improved heat and moisture exchange performance. The hygroscopic material content of the HME material may be at least 20%, be at least 30%, at least 40%, at least 50%, at least 60%, at least 70% or at least 80% by weight.

In cases in which the HME material is in the form of a sheet, the hygroscopic material content may be at least 40g/m², at least 60g/m², at least 80g/m², at least 100g/m², at least 120g/m², at least 140g/m², at least 160g/m², at least 180g/m² or at least 200g/m².

The HME material is preferably in the form of a sheet. The thickness of the sheet should preferably be great enough to retain heat and moisture while still providing sufficient surface area for heat and moisture exchange. In particular, the HME material preferably has a thickness of between 0.01 mm and 1.0 mm, more preferably between 0.05 mm and 0.5 mm, and most preferably between 0.1 mm and 0.3 mm and in particular about 0.2 mm.

The HME material is preferably in the form of elongate strips, which facilitates the formation of the HME material into HME media. The width of the HME material strip may be between 5 mm and 20 mm or between 8 mm and 18 mm, and for example may be about 9. 5mm or about 16 mm.

Discrete protrusions may extend from at least a part of two opposing surfaces of the HME material. First and second sides of a strip of HME material may thus both comprise discrete protrusions extend from their surface. The protrusions extending from a second surface of the HME material may be similar to any of the protrusions previously described, and may be similar to or different from the protrusions extending from the first surface.

The HME medium is preferably formed of multiple layers of HME material arranged adjacent to/overlying one another, and in particular is preferably a coil of HME material. The HME medium may be formed of five, four, three or two pieces of HME material, or one piece of HME material, formed into a coil. Alternatively, HME material could be folded back on itself repeatedly to form layers.

The height of the HME media corresponds to the width of the HME material used to form it, and hence is generally between 5mm and 20mm or between 8mm and 18mm, and for example may be about 9.5mm or about 16mm.

The diameter of the HME media depends on the thickness of the HME material, the number of revolutions in the coil and the separation between the layers of the coil. HME media are typically from 15mm to 80mm in diameter, and in particular may have a diameter of around 50mm in the case of relatively large HME media, or around 22mm in the case of relatively small HME media.

The HME device may be used to heat and humidify respiratory gases during tracheotomy or artificial ventilation with a respirator or anaesthesia apparatus. The HME device is preferably arranged such that substantially all gas flow through the device passes through the HME medium. The HME medium is preferably positioned such that gas flow through the device is substantially transverse to the plane of the HME material in the HME medium such that gas predominantly passes through the gaps between adjacent layers of the HME material.

The HME device preferably comprises a housing defining a heat and moisture exchange chamber containing the HME medium. The housing may further comprise two ports communicating between the heat and moisture exchange chamber and the exterior of the device. Each port preferably comprises standard connectors, such as Luer tapers, that are compatible with conventional breathing lines and associated apparatus. The ports are preferably located at opposite ends of the chamber and the chamber preferably has a greater transverse cross-sectional area than the ports.

The chamber preferably comprises a space adjacent to each port that is not occupied by the HME medium, which allows expansion of the gas upon entry into the chamber, such that gas flow is distributed substantially evenly throughout the HME medium.

The housing may be formed of any suitable material, but is preferably formed of injection moulded plastic. Some or all of the material of the housing may be transparent, or substantially transparent, to enable visual inspection of the HME medium.

The housing may comprise a sampling port for sampling gas in the chamber, which preferably includes a valve or closure for preventing gas flow through the sampling port during normal operation of the HME device.

There is provided a method of embossing an HME material comprising urging an HME material against an embossing surface, wherein the embossing surface comprises a plurality of projections.

The method may be performed such that the HME material is embossed with a pattern comprising a plurality of protrusions extending from at least part of one surface of the HME material. In particular, the method may be a method of manufacturing an HME material.

The integrity of the protrusions in the HME material is less susceptible to the effects of relative humidity at the time of their formation than corrugations. Accordingly, this method may be performed with less need for precise control of the relative humidity in the manufacturing environment. Cost savings can therefore be achieved compared to the known methods.

The HME material may be urged against the projections on the embossing surface by any suitable means. However, the HME material is preferably urged against the projections on the embossing surface by a second embossing surface. The second embossing surface may have a pattern of recesses that corresponds to the pattern of projections on the first embossing surface, which permit the projections to extend through the plane of the HME material. This permits greater deformation of the HME material by the projections and hence the formation of more prominent protrusions and perforations in the HME material.

The second embossing surface may further comprise projections that correspond to recesses provided in the first embossing surface. Both embossing surfaces may comprise both projections and recesses to provide protrusions extending from both sides of HME material during processing. The projections provided on the first embossing surface need not correspond in shape or distribution to the recesses provided on the first embossing surface. Similarly, the projections provided on the second embossing surface need not correspond in shape or distribution to the recesses provided on the second embossing surface. This allows the production of differently shaped and/or distributed protrusions on the opposing sides of HME material.

The first and second embossing surfaces may be the circumferential surfaces of first and second embossing rollers. The first and second embossing rollers may together define a nip at which the circumferential surfaces of the rollers come into contact. The projections of the first embossing roller may mesh with the recesses of the second embossing roller at the nip. Similarly, any projections provided on the second embossing roller may mesh with any recesses of the first embossing roller at the nip. The HME material may be passed through the nip by rotation of the embossing rollers, at which point the HME material is placed under sufficient pressure for the projections to generate protrusions and potentially also perforations in the HME material. This arrangement is particularly preferred as it facilitates the performance of the method as a continuous process by continuously passing HME material through the nip.

The projections of the embossing surface are preferably discrete projections and in particular are preferably completely surrounded by substantially non-projecting regions of the embossing surface.

The form and distribution of the protrusions formed in the HME material is primarily determined by the form and distribution of the projections on the embossing surface.

The pressure at which the HME material is urged against the embossing surface affects the amount of deformation of the HME material and hence the distance by which the protrusions protrude from the surface of the HME material. The projections may also perforate the HME material, such that the protrusions further comprise a perforation, if the HME material is urged against the embossing surface with sufficient pressure.

The height of the projections (ie the distance by which the projections extend from the embossing surface) may be at least 0.2mm, at least 0.4mm or at least 0.6mm, and may be less than 6.0mm, less than 4.0mm or less than 2.0mm. Accordingly, the height of the projections may be between 0.2mm and 6.0mm, between 0.4mm and 4.0mm or between 0.6mm and 2.0mm.

In particular, the height of the projections may be at least equal to, at least 2 times or at least 3 times thickness of the HME material, and may be less than 30 times, less than 20 times or less than 10 times the thickness of the HME material. Accordingly, the height of the projections may be between 1 and 30 times, between 2 and 20 times or between 3 and 10 times the thickness of the HME material.

Relatively short projections, such as projections having a height of less than 1.0mm, 0.8mm or 0.6mm, or less than 5 times, 4 times or 3 times the thickness of the HME material, are generally more suitable for creating protrusions that do not comprise perforations. Relatively long projections, such as projections having a height of greater than 1.0mm, 1.2mm or 1.4mm, or greater than 5 times, 6 times or 7 times the thickness of the HME material, are generally more suitable for creating protrusions that comprise perforations in the HME material.

The cross-section of the projections may be any of a wide variety of shapes, such as circular, elliptical, square, rectangular, rhomboid or any combination of these. The cross-section of the projections may have a maximum dimension of at least 0.4 mm, at least 0.6 mm or at least 0.8 mm, and no more than 5.0 mm, no more than 3.0 mm or no more than 2.0 mm. In particular, the cross-section of the projections may have a maximum dimension of between 0.4 mm and 5.0 mm, between 0.6 mm and 3.0 mm or between 0.8 mm and 2.0 mm. In particular, where the cross-section of the projections is circular, the maximum dimension corresponds to the diameter of each projection.

The projections may form perforations in the HME material by punching through the material, although this typically produces relatively large quantities of swarf in the form particles of the HME material. The projections may also form perforations in the HME material by cutting, which tends to produce less swarf.

The projections may be any of a wide variety of shapes, such as hemispherical, frustoconical, cylindrical, pyramidal, cuboid, hemicylindrical or any combination of these.

Projections may be in the form of cylinder having a hemispherical end, in which the cylinder may be relatively short (ie having a diameter that is greater than its height) or relatively long (ie having a height that is greater than its diameter). Such projections may also form protrusions comprising perforations in the HME material, especially when projections are relatively long and/or small in diameter.

The projections may also be in the form of a cylinder in which the surface of the distal end is inclined relative to its longitudinal axis. In particular, the surface of the distal end may be inclined at between 30 and 60 degrees, or about 45 degrees relative to its longitudinal axis. Accordingly, the distal end of the projection forms a point that is able to pierce the HME material and form a perforation in the HME material in the form of a generally C-shaped cut, such that a substantially circular flap is formed.

The embossing surfaces are preferably formed of a material that is sufficiently hard to enable effective embossing of the HME material. Particularly suitable materials for forming the patterned surface are metals. A preferred metal is steel, such as stainless steel.

The method may include a step of reducing the moisture content of the HME material prior to embossing, particularly where a hygroscopic additive is used. The HME material may, for example, be exposed to an environment of low humidity, or heated, for example by passing the HME material over a heated surface, such as the surface of a roller, or by exposing it to infra-red radiation. HME material is preferably free or substantially free from microbial contamination. This may be achieved by carrying out the method in a sterile or aseptic environment. Alternatively, the method may comprise an additional step sterilising the HME material, for example by gamma irradiation or exposure of the HME material to ethylene oxide.

The method may further comprise a step of forming the HME material into an HME medium, such as an HME medium. In particular, this may comprise attaching one or more pieces of the HME material to a spindle and rotating the spindle such that the HME material is wound into a coil around the spindle. The resulting HME medium may then be removed from the spindle and the process repeated.

The method may further comprise introducing the HME medium into a housing in order to produce an HME device.

The invention has the potential to provide filters, especially larger filters, having a lower filter weight, ie requiring a shorter length of material for same diameter coil. This provides cost savings and also potentially lower resistance to flow, both of which are advantageous.

Wherever practicable, any of the essential or preferable features defined in relation to any one aspect may be applied to any further aspect. Accordingly, the invention may comprise various alternative configurations of the features defined above.

A currently preferred embodiment of the invention will now be described, by way of illustration only, with reference to the accompanying drawings, in which:
Figure 1 is a schematic representation, not to scale, of an apparatus for manufacturing HME media;
Figure 2A is a front view of a first embodiment of an upper embossing roller of the apparatus of Figure 1;
Figure 2B is a side view of the upper embossing roller of Figure 2A with a partial cutaway depicting the internal structure of the roller;
Figure 3A is a front view of a second embodiment an upper embossing roller of the apparatus of Figure 1;
Figure 3B is a side view of the upper embossing roller of Figure 3A with a partial cutaway depicting the internal structure of the roller;
Figure 4A is a front view of a first embodiment of a lower embossing roller of the apparatus of Figure 1;
Figure 4B is a side view of the lower embossing roller of Figure 4A.
Figure 5A is a front view of a second embodiment of a lower embossing roller of the apparatus of Figure 1;
Figure 5B is a side view of the lower embossing roller of Figure 5A.
Figures 6A is a front view of a third embodiment of a lower embossing roller of the apparatus of Figure 1;
Figure 6B is a side view of the lower embossing roller of Figure 6A.
Figures 7A is a front view of a fourth embodiment of a lower embossing roller of the apparatus of Figure 1;
Figure 7B is a side view of the lower embossing roller of Figure 7A.
Figure 8 is a perspective view of an embossing assembly;
Figure 9 is a cross-sectional view, schematic and not to scale, of the embossing assembly of Figure 8 immediately before the HME material passes between the upper and lower embossing rollers;
Figure 10A is a plan view, schematic and not to scale, of an embossed HME material;
Figure 10B is a cross-sectional view taken along line A-A of a first embodiment of the embossed HME material of Figure 10A;
Figure 10C is a cross-sectional view taken along line A-A of a second embodiment of the embossed HME material of Figure 10A;
Figure 10D is a cross-sectional view taken along line A-A of a third embodiment of the embossed HME material of Figure 10A;
Figures 11A-C are plan views, schematic and not to scale, of further embodiments of embossed HME material;
Figure 12A is a side view of an HME medium in the form of a coil of embossed HME material;
Figure 12B is a perspective view of the HME medium of Figure 12A in a partially unwound configuration;
Figure 13A is a plan view, schematic and not to scale, of an embodiment of an embossed HME material;
Figure 13B is a cross-sectional view of the embossed HME material of Figure 13A;
Figure 13C is a plan view, schematic and not to scale, of a further embodiment of an embossed HME material;
Figure 13D is a cross-sectional view of the embossed HME material of Figure 13C;
Figure 14 is a side view of a cutaway section of a further embossing roller, schematically depicting the internal structure of the roller; and,
Figure 15 is a cross-sectional view of a further example of embossed HME material.

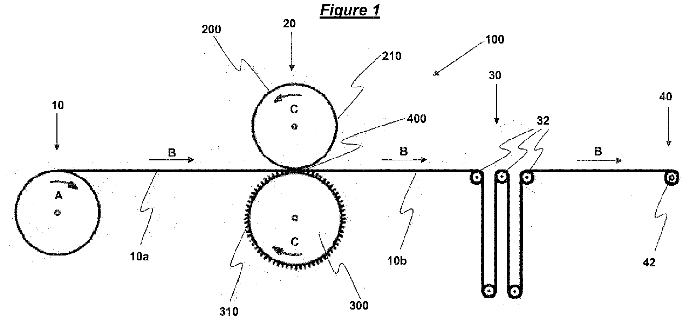

Figure 1 depicts an apparatus for manufacturing HME media 100. The apparatus 100 comprises a source reel 10 carrying HME material 10a, an embossing or cutting assembly 20, a tension control assembly 30, and a winding assembly 40.

The HME material 10a is in the form of a strip that is unwound by the rotation of the source reel 10 in the direction of arrow A and transferred along the apparatus 100 in the direction of arrow B towards the embossing assembly 20.

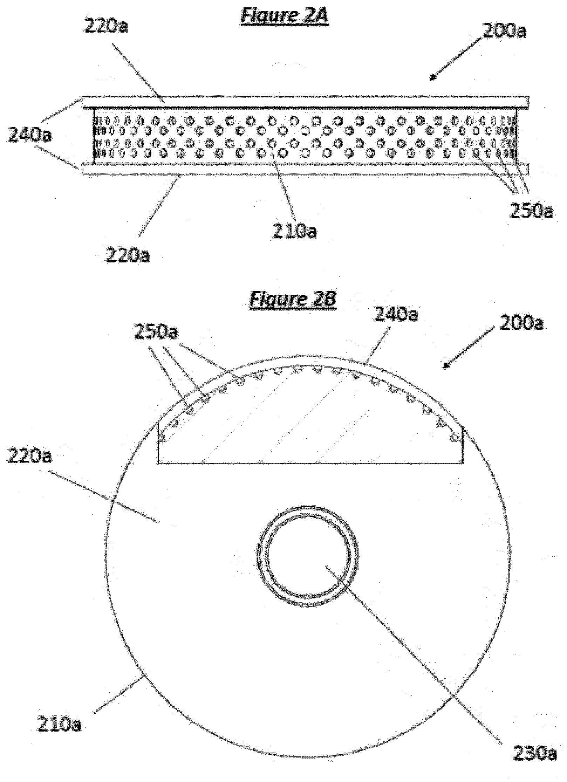

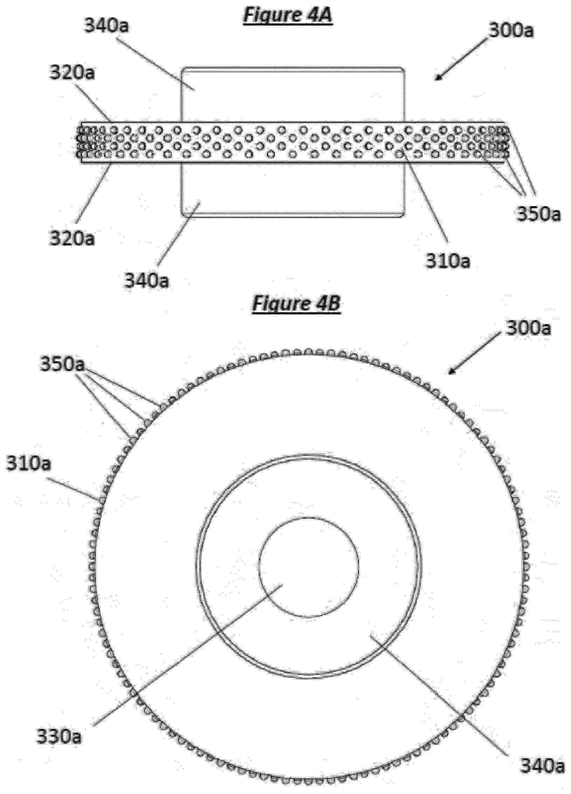

The embossing assembly 20 comprises an upper embossing roller 200 and a lower embossing roller 300 that together define a nip 400. A motor (not shown) drives the rotation of the embossing rollers 200,300 in the direction or arrows C. The circumferential surface 210 of the upper embossing roller 200 comprises a pattern of recesses (see, for example, Figures 2A and 2B) while the circumferential surface 310 of the lower embossing roller 300 comprises a corresponding pattern of projections (see, for example, Figures 4A and 4B). The recesses of the upper embossing roller 200 mesh with the projections of the lower embossing roller 300 at the nip 400.

The HME material 10a is drawn into the nip 400 by the rotation of the embossing rollers 200,300 in the direction of arrows C. As the HME material 10a passes through the nip 400 it is compressed between the meshing recesses and projections on the circumferential surfaces 210,310 of the embossing rollers 200,300, thereby producing an embossed pattern on the HME material 10b. The embossed pattern comprises a plurality of protrusions corresponding to the pattern of recesses and projections on the circumferential surfaces 210,310 of the embossing rollers 200,300.

The embossed HME material 10b passes out of the nip 400 and enters the tension control assembly 30, which comprises a number of rollers 32 that control the tension of the HME material 10a, 10b as it passes through the nip 400.

The winding assembly comprises a spindle 42 to which the embossed HME material 10b is attached. The spindle 42 is rotated to wind the embossed HME material 10b into a coil as it passes out of the tension control assembly 30. When sufficient embossed HME material 10b has been wound into the coil to form an HME medium, the embossed HME material 10b is cut and the HME medium is removed from the spindle 42. The free end of the embossed HME material 10b is then attached to the spindle 42 and the process repeated. However, in alternative embodiments, the embossed HME material 10b may be wound directly onto a roller after passage through the tension control assembly 30 and processed into HME media in a separate process.

The embossed pattern maintains separation between the adjacent layers of the HME material 10b in HME media, thereby improving the heat and moisture exchange performance of the HME media and reducing the resistance of the HME media to gas flow. The HME media may be introduced into a housing in order to produce an HME device.

The integrity of the embossed pattern and hence its ability to maintain separation between the adjacent layers of the HME material 10b in HME media is less susceptible to relative humidity during manufacture and hence this method is capable of producing HME media with more consistent performance without the need to control humidity during manufacture.

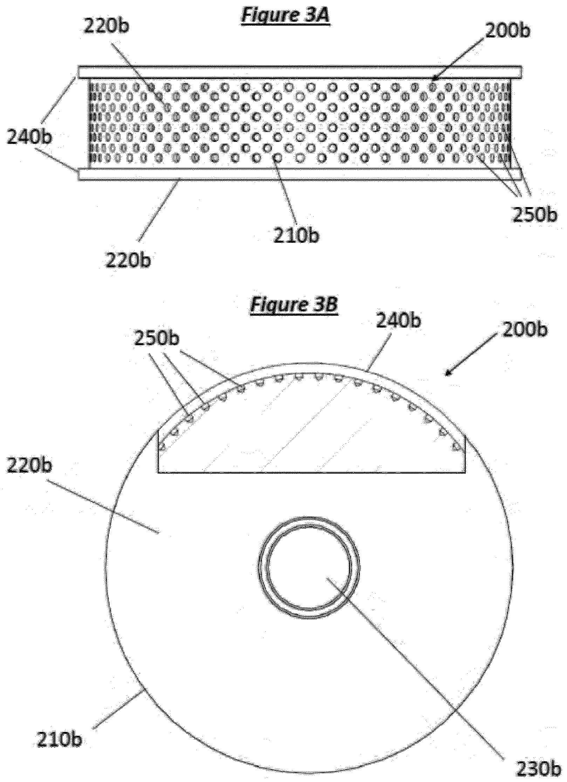

Figures 2A and 2B depict a first embodiment of upper embossing roller 200a and Figures 3A and 3B depict a second embodiment of an upper embossing roller 200b. The upper embossing rollers 200a,200b are generally disk-shaped and each comprise a circumferential surface 210a,210b, two generally circular faces 220a,220b, a channel 230a,230b passing between the centres of the circular faces 220a,220b, and ridges 240a,240b extending radially from both edges of the circumferential surfaces 210a,210b.

The circumferential surface 210a of the first embodiment of the upper embossing roller 200a (Figures 2A and 2B) comprises a pattern of generally cylindrical recesses 250a. The recesses 250a are arranged in four staggered rows extending around the circumference of the roller 200a.

The circumferential surface 210b of the second embodiment of the upper embossing roller 200b (Figures 3A and 3B) has a greater width than that of the first embodiment of the upper embossing roller 200a. The circumferential surface 210b also comprises a pattern of generally cylindrical recesses 250b, which in this embodiment are arranged in seven rather than four staggered rows extending around the circumference of the roller 200b.

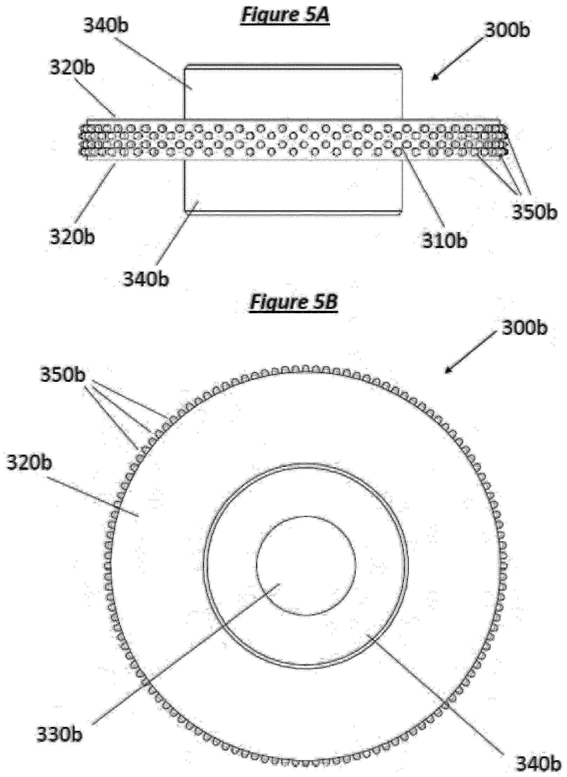

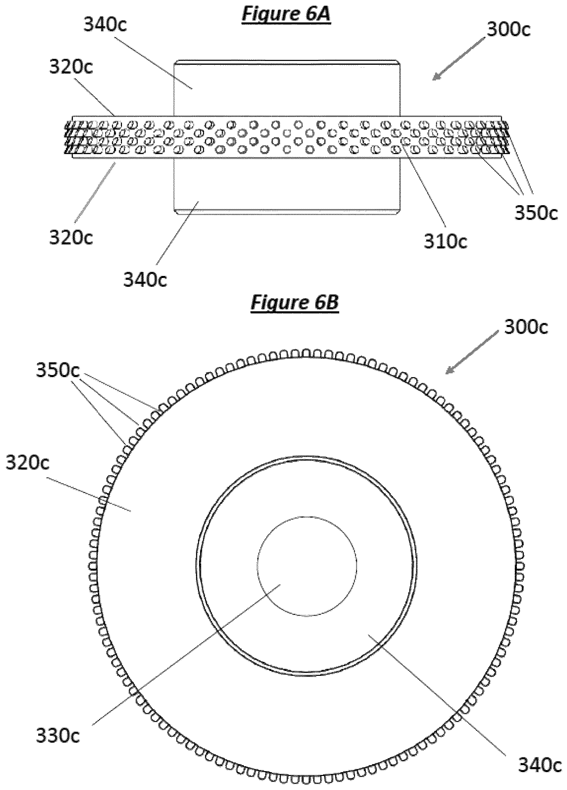

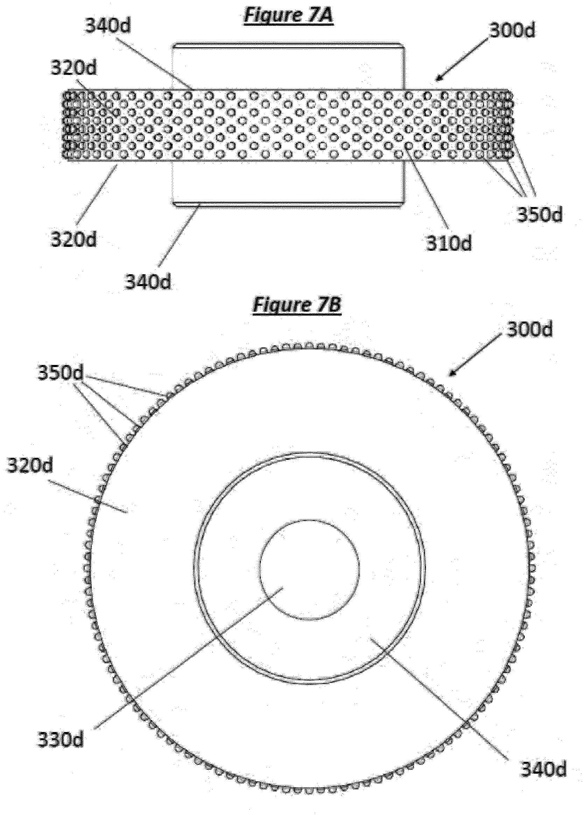

Figures 4A and 4B depict a first embodiment of a lower embossing roller 300a, Figures 5A and 5B depict a second embodiment of a lower embossing roller 300b, Figures 6A and 6B depict a third embodiment of a lower embossing roller 300c, and Figures 7A and 7B depict a fourth embodiment of a lower embossing roller 300d. The lower embossing rollers 300a,300b,300c,300d are also generally disk-shaped and each comprises a circumferential surface 310a,310b,310c,310d, two generally circular faces 320a,320b,320c,320d, a channel 330a,330b,330c,330d passing between the centres of the circular faces 320a,320b,320c,320d, and annular collars 340a,340b,340c,340d that surround each end of the channel 330a,330b, 330c,330d on each circular face 320a,320b,320c,320d.

The circumferential surfaces 310a,310b,310c,310d of the lower embossing rollers 300a,300b,300c,300d comprises patterns of projections 350a,350b,350c,350d. The projections 350a of the first embodiment of a lower embossing roller 300a (Figures 4A and 4B) are in the form of a relatively short cylinder (ie the diameter of the cylinder is significantly greater than its height) having a hemispherical end. The projections 350a are arranged in four staggered rows extending around the circumference of the roller.

The projections 350b of the second embodiment of a lower embossing roller 300b (Figures 5A and 5B) are in the form of a relatively long cylinder (ie the height of the cylinder is significantly greater than its diameter) having a hemispherical end. The projections 350b are arranged in four staggered rows extending around the circumference of the roller 300b.

The projections 350c of the third embodiment of a lower embossing roller 300c (Figures 6A and 6B) are in the form of a cylinder having a top surface inclined at 45 degrees to its longitudinal axis. The projections 350b are arranged in four staggered rows extending around the circumference of the roller 300c.

The circumferential surface 310d of the fourth embodiment of the lower embossing roller 300d (Figures 7A and 7B) has a greater width than the circumferential surfaces 310a, 310b,310c of the other embodiments of the upper embossing roller 300a,300b, 300c. The projections 350d are in the form of relatively short cylinders (ie the diameter of each cylinder is significantly greater than its height) having a hemispherical end, and are similar in form to the projections 350a of the first embodiment of a lower embossing roller 300a. The projections 350d are arranged in seven rather than four staggered rows extending around the circumference of the roller 300d.

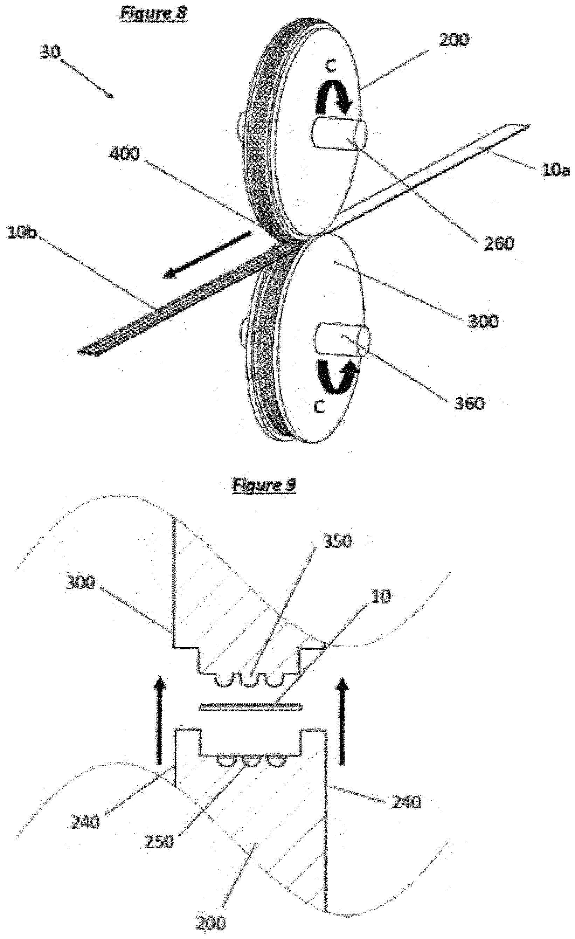

Referring now to Figures 8 and 9, the embossing rollers 200,300 engage the apparatus for manufacturing HME media 100 via axles 260,360 that pass through the channels 230,330. The rotation of the embossing rollers 200,300 may be driven by a motor (not shown) acting upon one or both axles 260,360. The recesses 250 of the upper embossing roller 200 have the same distribution as, and are of sufficient size to accommodate, the projections 350 of the lower embossing roller 300, and hence are able to mesh at the nip 400 as the embossing rollers 200,300 are rotated.

A precise fit between the recesses 250 and projections 350 is not necessarily required and hence it is possible for the same upper embossing roller 200 to be used in conjunction with different lower embossing rollers 300. In particular, the distribution of the recesses 250a in the first embodiment of the upper embossing roller 200a is the same as the distribution of the projections 350a,350b,350c in the first, second and third embodiments of the lower embossing rollers 300a,300b, 300c, and hence any of these lower embossing rollers may be used in conjunction with this upper embossing roller. This arrangement may be used to emboss a narrower HME material.

In addition, the distribution of the recesses 250b in the second embodiment of the upper embossing roller 200b is the same as the distribution of the projections 350d on the fourth embodiment of the lower embossing rollers 300d and hence these lower and upper embossing rollers may be used in conjunction. This arrangement may be used to emboss a broader HME material.

The HME material 10a is drawn into the nip 400 by the rotation of the embossing rollers 200,300 in the direction of arrows C and is guided onto the circumferential surfaces 210,310 and into contact with the projections 350 by the ridges 240. As the HME material 10a is drawn into the nip 400, the projections 350 apply increasing pressure to the HME material 10a, thus creating indentations in the lower surface of the HME material 10a and corresponding protrusions on the upper surface of the HME material 10a. The presence of the recesses 250 on the upper embossing roller permits the projections 350 to extend through the plane of the HME material 10a at the point at which maximum pressure is applied to the HME material at the nip 400. This permits greater deformation of the HME material 10a by the projections 350 and hence the formation of more prominent protrusions, and potentially also perforations, in the HME material 10a by the projections 350.

The pressure applied to the HME material 10a at the nip 400 should be sufficient to form a protrusion or perforation in the paper. It will be appreciated that the specific pressure required may vary, depending on the thickness and composition of a particular HME material, but can be readily determined by a skilled person. It is envisaged that the operating pressure provided at the nip 400 will be similar regardless of whether protrusions or perforations are required. Different pin sizes and geometries will nonetheless result in different pressures ultimately being applied to the material.

The height or length of projections, for example, can have a relatively significant influence on the ultimate pressure applied to the HME material. More tension will be created in HME material drawn through a nip including longer protections, and so the pressure applied to the material will effectively increase. Similarly, a larger number or denser pattern of projections will similarly increase the tension in the material and, thus, the ultimate pressure applied. By controlling these factors, the invention allows some control of applied pressure without the need to adjust the pressure at the nip 400.

The HME material 10b that is ejected from the nip 400 by the rotation of the embossing rollers 200,300 in the direction of arrows C thus carries an embossed pattern comprising a plurality of protrusions. The form and distribution of the protrusions is determined by the form and distribution of the projections 350 on the lower embossing roller 30 as well as the level of pressure that is applied to the HME material at the nip 400. Accordingly, a wide variety of embossed patterns can be formed by this method with the use different embossing rollers 200,300 and the application of different pressures at the nip 400.

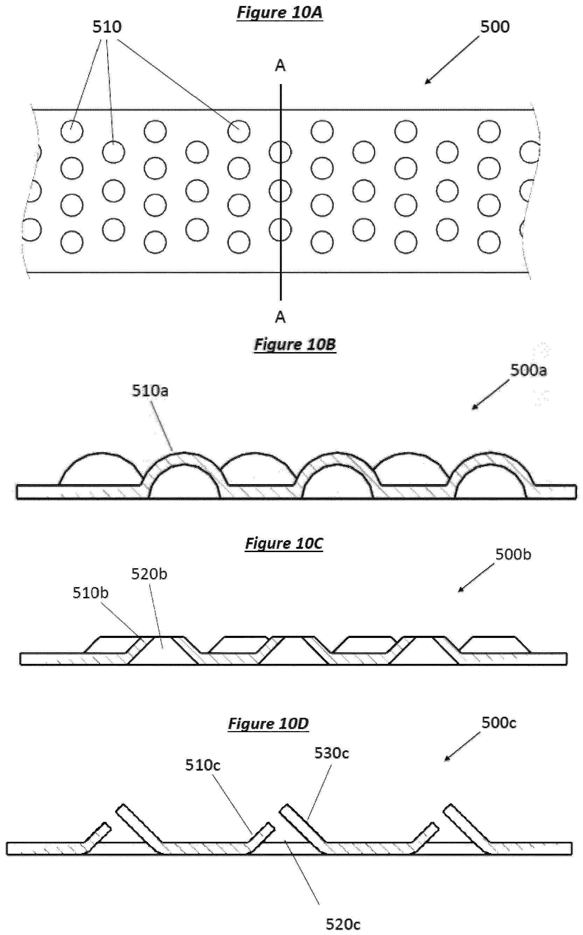

Figure 10A depicts an HME material 500 having an embossed pattern formed of a plurality of protrusions 510. The protrusions 510 are generally circular in plan view and are arranged regularly in seven offset rows extending longitudinally along the HME material 500.

Figure 10B is a cross-sectional view of a first embodiment of the HME material 500a depicted in Figure 10A taken along line A-A in which the protrusions 510a are generally hemispherical in shape and the HME material 500a is not perforated. The protrusions 510a may be formed by projections in the form of a relatively short cylinder having a hemispherical end, such as those of the first embodiment of the lower embossing roller (Figures 4A and 4B).

Figure 10C is a cross-sectional view of a second embodiment of the HME material 500b depicted in Figure 10A taken along line A-A in which the protrusions 510b are generally frustoconical in shape and comprise perforations 520b in the HME material 500b at their apex. The protrusions 510b may be formed by generally 'bullet-shaped' projections, for example in the form of a relatively long spiked cylinder. Such projections are able to punch through the HME material 500b when sufficient pressure is applied at the nip 400. This has been found to produce a significant amount of swarf in the form of particles of HME material.

Figure 10D is a cross-sectional view of a third embodiment of the HME material 500c depicted in Figure 10A taken along line A-A in which the protrusions 510c are in the form of a flap 530c extending from one side of a perforation 520c. The protrusions 510c may be formed by projections in the form of a cylinder having a hemispherical end, such as those of the second embodiment of the lower embossing roller (Figures 5A and 5B). Alternatively, cylinders having a top surface inclined at 45 degrees to its longitudinal axis, such as those of the third embodiment of the lower embossing roller 300c (Figures 6A and 6B), could be used. Such projections are able to cut into the HME material 500c as pressure is applied at the nip 400 in order to produce the perforations 520c and flaps 530c. This has been found to produce a cleaner cut with less swarf than perforation with longer hemispherical projections of the type found on the second embodiment of the lower embossing roller (Figures 5A and 5B), where the perforations are typically formed by a tearing action.

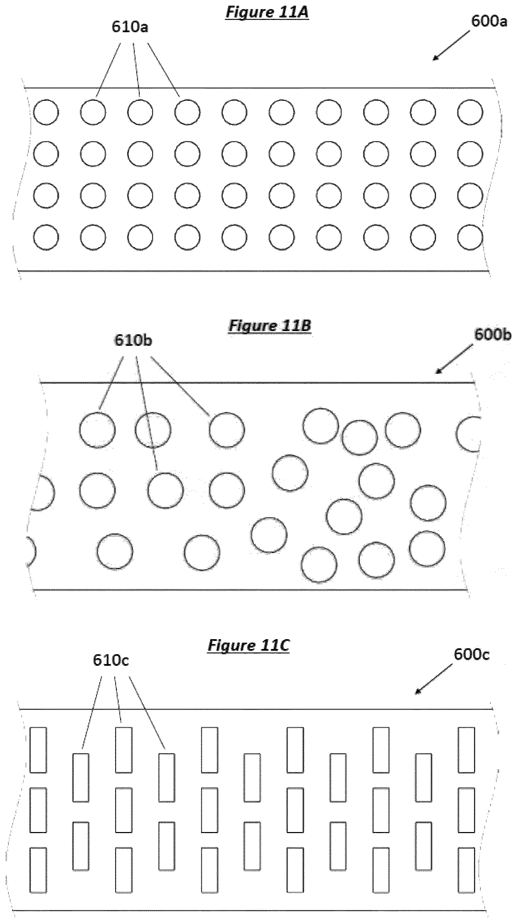

Figures 11A-C depict further embodiments of an embossed HME material 600a, 600b, 600c with an embossed pattern comprising a plurality of protrusions 610a, 610b, 610c. The embodiment depicted in Figure 11A comprises generally circular protrusions arranged regularly in four non-offset rows. The embodiment depicted in Figure 11B comprises generally circular protrusions arranged irregularly. The embodiment depicted in Figure 11C comprises generally rectangular protrusions arranged regularly in five offset rows.

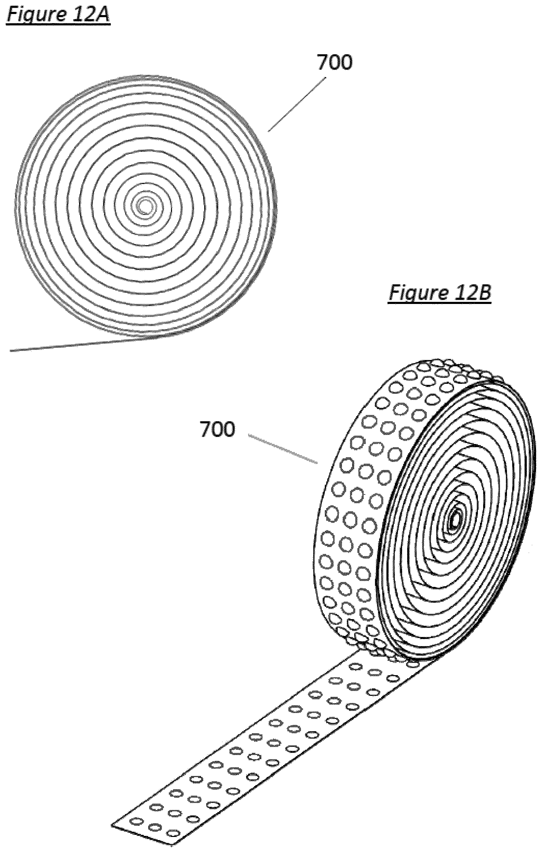

Figures 12A and 12B depict an HME medium 700 in the form of a coil of embossed HME material. Figure 12B indicates that the HME material from which the HME medium 700 is formed comprises an embossed pattern in the form of generally circular indentations 710 arranged regularly in three non-offset rows. The protrusions 710 maintain the separation between the adjacent layers of the HME material in the HME medium 700, which improves the heat and moisture exchange performance of the HME medium 700 and reduces the resistance of the HME medium 700 to gas flow.

Protrusions produced by the method described above maintain the separation between the adjacent layers of the HME material even when the HME material is placed under a relatively high level of tension, such as when the HME material is wound into a relatively tight coil. This is particularly significant for relatively small HME media in which the HME material must be wound more tightly in order to prevent the formation of an opening at the centre of the HME media, which would create a passage through which gas would be able to flow without being adequately exposed to the HME material.

### Example 1 - Embossing a 9.5mm wide strip of HME material using the lower embossing roller depicted in Figures 4A and 4B

An HME material was embossed using an embossing assembly substantially as discussed above with reference to Figure 8, using an upper embossing roller substantially as discussed above with reference to Figures 2A, 2B and a lower embossing roller substantially as discussed above with reference to Figures 4A and 4B. The embossing rollers had a diameter of 69.95mm and circumferential surfaces with a width of 9.5mm.

The HME material was a continuous strip having a width of 9.5mm and a thickness of 0.2mm, and was formed of cellulose based paper containing cotton fibres. However, this embossing method is equally applicable to other forms of HME material, including HME materials containing any form of natural or synthetic fibres and hygroscopic additives such as CaCl.

The projections of the lower embossing roller were in the form of a relatively short cylinder (ie the diameter of the cylinder is significantly greater than its height) having a hemispherical end (see Figures 4A and 4B). The apex of each projection extended 0.7mm from the circumferential surface of the lower embossing roller.

The HME material was passed through the nip between the embossing rollers in order to produce an embossed pattern made up of a plurality of substantially hemispherical protrusions (see Figure 10B) on one surface of the HME material. The protrusions were arranged in four offset rows extending along the length of the strip of HME material.

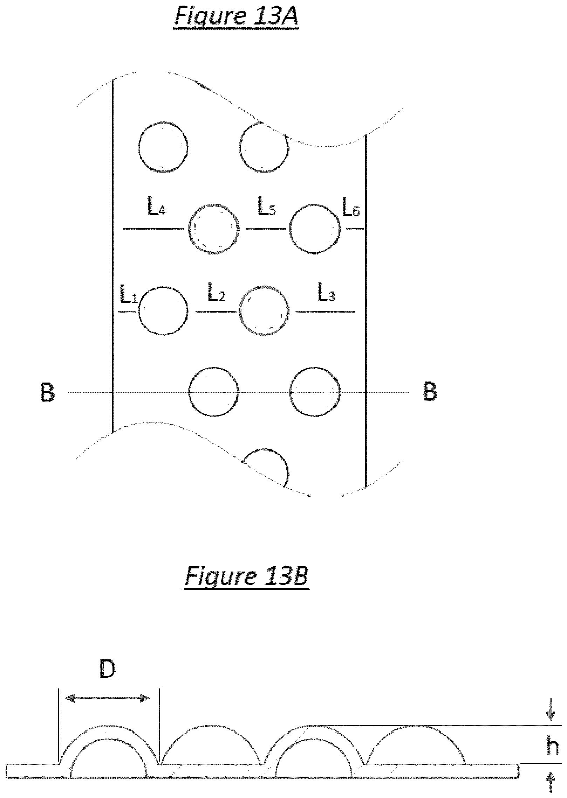

Figures 13A and 13B are schematic representations of an embodiment of an embossed HME material having four offset rows of protrusions, on which measurements relating to the separation between the protrusions (L1-L6), and the diameter (D) and height (h) of the protrusions are marked. With reference to Figures 13A and 13B, the average dimensions and separation between the protrusions was:

| | |
|---|---|
| L₁ | 0.79mm |
| L₂ | 2.25mm |

| | |
|---|---|
| L₃ | 2.92mm |
| L₄ | 2.92mm |
| L₅ | 2.28mm |
| L₆ | 0.97mm |
| D | 1.85mm |
| h | 0.45mm |

It will be understood that various known methods, such as shadowgraphy, can be used to measure these dimensions.

All protrusions were formed under substantially identical conditions although their height (h) ranged from 0.41 mm to 0.51 mm. This variation is believed to be indicative of the tolerance of the HME material when forming protrusions and possibly arises due to small changes in pressure depending on the unique conditions at the nip.

A strip of the embossed HME medium of 73cm (+/- 10mm) in length was then wound into a coil in order to form an HME medium for inclusion into an HME device.

### Example 2 - Embossing a 9.5mm wide strip of HME material using the lower embossing roller depicted in Figures 5A and 5B

The HME material was also embossed using the protocol of Example 1 in which the lower embossing roller was substantially as depicted in Figures 5A and 5B. This lower embossing roller also had a diameter of 69.95mm and circumferential surfaces with a width of 9.5mm. The projections were in the form of a relatively long cylinder (ie the height of the cylinder is significantly greater than its diameter) having a hemispherical end (see Figures 5A and 5B). The apex of each projection extended 1.5mm from the circumferential surface of the lower embossing roller.

The HME material was passed through the nip between the embossing rollers in order to produce an embossed pattern made up of a plurality of protrusions on one surface of the HME material that were generally frustoconical in shape, similar to those shown in Figures 10B or 10C, but with flaps of material remaining at their apex as in Figure 10D). The protrusions were arranged in four offset rows extending along the length of the strip of HME material.

The dimensions and distribution of the protrusions was measured at five locations along the embossed strip of HME material using the method used in Example 1. With reference to Figures 13A and 13B, the average dimensions and separation between the protrusions was:

| | |
|---|---|
| L₁ | 1.64mm |
| L₂ | 3.09mm |
| L₃ | 2.96mm |
| l_₄ | 3.65mm |
| L₅ | 3.10mm |
| L₆ | 0.94mm |
| D | 1.03mm |
| h | 0.62mm |

### Example 3 - Embossing a 9.5mm wide strip of HME material using the lower embossing roller depicted in Figures 6A and 6B

The HME material was also embossed using the protocol of Example 1 in which the lower embossing roller was substantially as depicted in Figures 6A and 6B. This lower embossing roller also had a diameter of 69.95mm and circumferential surfaces with a width of 9.5mm. The projections of the lower embossing roller were in the form of a cylinder having a top surface inclined at 45 degrees to its longitudinal axis (see Figures 6A and 6B). The apex of each projection extended 1.3mm from the circumferential surface of the lower embossing roller.

The HME material was passed through the nip between the embossing rollers in order to produce an embossed pattern made up of a plurality of protrusions in the form of a flap extending from one side of a perforation (see Figure 10D). The protrusions were arranged in four offset rows extending along the length of the strip of HME material.

The dimensions and distribution of the protrusions was measured at five locations along the embossed strip of HME material using the method used in Example 1. With reference to Figures 13A and 13B, the average dimensions and separation between the protrusions was:

| | |
|---|---|
| L₁ | 1.30mm |
| L₂ | 3.10mm |
| L₃ | 3.29mm |
| L₄ | 3.30mm |
| L₅ | 3.05mm |
| L₆ | 1.27mm |
| D | 0.97mm |
| h | 0.58mm |

### Example 4 - Embossing a 16mm wide strip of HME material using the lower embossing roller depicted in Figures 7A and 7B

A strip of the HME material 16mm rather than 9.5mm wide was also embossed using the protocol of Example 1 in which the lower embossing roller was substantially as depicted in Figures 7A and 7B. The lower embossing roller had a diameter of 69.95mm and a circumferential surface having a width of 16mm and carrying projections arranged in seven offset rows extending around the circumference of the roller. The projections were in the form of relatively short cylinders (ie the diameter of the cylinder is significantly greater than its height) having hemispherical ends (see Figures 7A and 7B). The apex of each projection extended 0.7mm from the circumferential surface of the roller.

An upper embossing roller substantially as depicted in Figures 3A and 3B having a 16mm wide circumferential surface and recesses arranged in seven offset rows extending around the circumference of the roller was used in combination with this upper embossing roller.

The HME material was passed through the nip between the embossing rollers in order to produce an embossed pattern made up of a plurality of substantially hemispherical protrusions on one surface of the HME material. The protrusions were arranged in seven offset rows extending along the length of the strip of HME material.

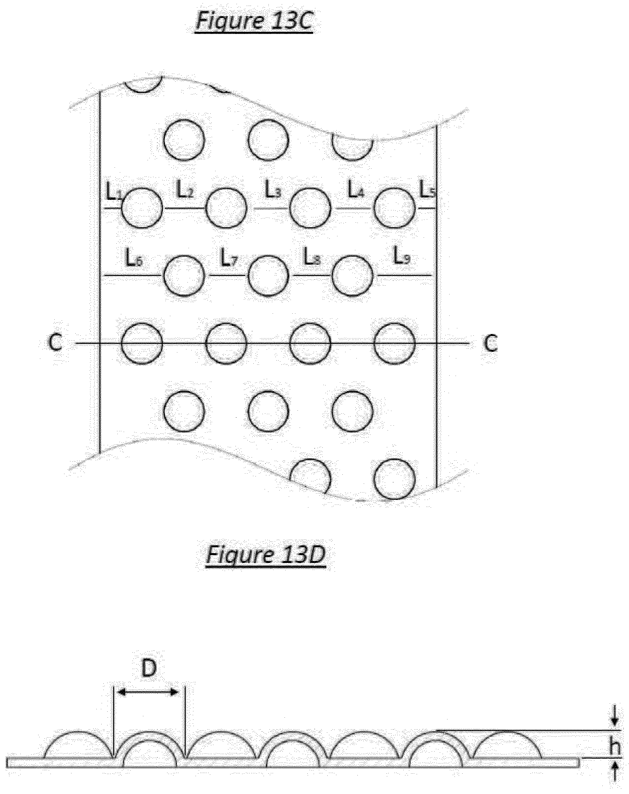

The dimensions and distribution of the protrusions was measured at five locations along the HME material using the method used in Example 1. Figures 13C and 13D are schematic representations of an embodiment of an embossed HME material having seven offset rows of protrusions, on which measurements relating to the separation between the protrusions (L1-L9), and the diameter (D) and height (h) of the protrusions are marked. With reference to Figures 13C and 13D, the average dimensions and distribution of the protrusions was:

| | |
|---|---|
| L₁ | 1.41mm |
| L₂ | 3.33mm |
| L₃ | 3.31 mm |
| L₄ | 3.28mm |
| L₅ | 1.84mm |
| L₆ | 3.37mm |
| L₇ | 3.29mm |
| L₈ | 3.25mm |
| L₉ | 3.87mm |
| D | 0.76mm |
| h | 0.44mm |

A small difference in average protrusion height can be seen in comparison to example 1 above (example 1 - 0.45mm, example 4 - 0.43mm), which would indicate a difference in pressure despite the similar conditions and projections used in both examples. This could be a result of different projection distribution/patterns, and/or the wider paper may result in a slightly lower material tension at the nip, even if the tension control assembly was the same in both examples, leading to a lower applied pressure in example 4.

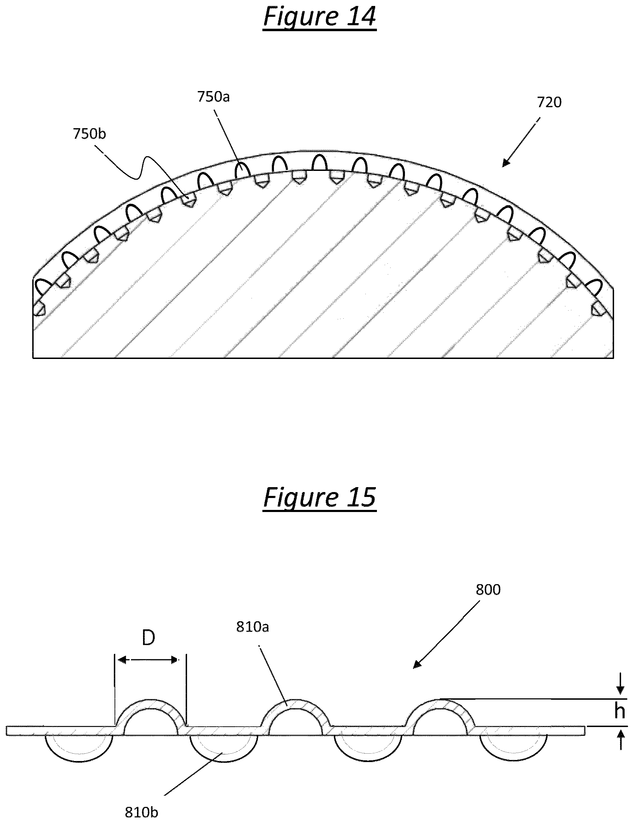

It will be understood that the invention is not limited to the specific examples described above. For example, an embodiment is considered where both upper and lower rollers contain both projections and depressions, so that protrusions are formed on both sides of the paper. An example of this type of roller is illustrated in Figure 14, which shows a cutaway section 720 of a roller similar to that illustrated in Figure 3B, but with raised protrusions750a provided alternately with the recesses 750b. It will be understood that providing a pair of such rollers allows the creation of protrusions from opposite sides of a paper or other material simultaneously. Figure 15 shows an example of a section of HME material 800 with protrusions 810a,810b projecting, respectively, from the upper and lower surfaces as shown. The dimensions D and h may be of similar magnitude to the examples already described herein.

Although introducing some additional complexity, for example in gear timing and design, providing protrusions from both sides of HME material may allow even greater control of the properties of filters formed using such material.

## Claims

1. An HME material (500,600a,600b,600c,800) for use in heat and moisture exchange with respiratory gases comprising a plurality of discrete protrusions (510a,510b,510c,610a,610b,610c,810a,810b) extending from at least part of one surface of the HME material (500,600a,600b,600c,800), **characterised in that** each protrusion (510a,510b,510c,610a,610b,610c,810a, 810b) is completely surrounded by substantially non-protruding HME material.

2. An HME material (500,600a,600b,600c,800) according to Claim 1, wherein the protrusions (510a,510b,510c,610a,610b,610c,810a,810b) do not span the entire width of the HME material (500,600a,600b,600c,800).

3. An HME material (500,600a,600b,600c,800) according to Claim 1 or 2, wherein the protrusions (510a,510b,510c,610a,610b,610c,810a,810b) are hemispherical, frustoconical, cylindrical, pyramidal, cuboid, hemicylindrical or any combination of these.

4. An HME material (500,600a,600b,600c,800) according to any preceding claim, wherein the density of the protrusions (510a,510b,510c,610a,610b,610c, 810a,810b) per cm² is between 3 and 50, between 5 and 40, between 8 and 30 or between 10 and 20.

5. An HME material (500,600a,600b,600c,800) according to any preceding claim, wherein each protrusion (510b,510c) comprises a perforation (520b,520c) in the HME material.

6. An HME material (500,600a,600b,600c,800) according to Claim 5, wherein the perforations (520c) are cuts in the HME material that partially surround a region of HME material, such that that region of HME material forms a flap (530c) extending from the surface of the HME material.

7. An HME material (500,600a,600b,600c,800) according to any preceding claim, wherein the HME material (500,600a,600b,600c,800) comprises cellulose paper.

8. An HME material (500,600a,600b,600c,800) according to any preceding claim, wherein discrete protrusions (510a,510b,510c,610a,610b,610c,810a,810b) extend from at least a part of two opposing surfaces of the HME material.

9. An HME medium (700) comprising a plurality of overlying layers of an HME material (500,600a,600b,600c,800) according to any preceding claim.

10. The HME medium (700) of Claim 9, wherein the HME medium is in the form of a coil of the HME material (500,600a,600b,600c,800).

11. An HME device comprising a HME medium (700) according to Claim 9 or 10.

12. A method of embossing an HME material (500,600a,600b,600c,800) for use in heat and moisture exchange with respiratory gases comprising urging an HME material against an embossing surface (310a,310b, 310c,310d) comprising a plurality of discrete projections (350a,350b,350c,350d, 750a), **characterised in that** each discrete projection (350a,350b,350c,350d,750a) of the embossing surface (310a,310b,310c,310d) is completely surrounded by substantially non-projecting regions of the embossing surface.

13. The method of Claim 12, wherein the HME material is urged against the projections (350a,350b,350c,350d,750a) on the embossing surface (310a,310b, 310c,310d) by a second embossing surface (210a,210b) comprising recesses (250a,250b,750b) that correspond to the projections (350a,350b,350c,350d,750a) of the first embossing surface (310a,310b,310c,310d).

14. The method of Claim 13, wherein the second embossing surface further comprises projections (750a) that correspond to recesses (750b) provided in the first embossing surface.

15. The method of any of Claim 12 to 14, wherein the projections (350a,350b, 350c,350d,750a) form perforations in the HME material (500,600a,600b,600c, 800).

## Patentansprüche

1. HME-Material (500,600a,600b,600c,800) zur Verwendung bei Wärme- und Feuchtigkeitsaustausch mit Atemgasen, umfassend eine Vielzahl von einzelnen Vorsprüngen (S10a,510b,510c,610a,610b,610c,810a,810b), die sich von zumindest Teil einer Oberfläche des HME-Materials (500,600a,600b,600c,800) erstreckt, **dadurch gekennzeichnet, dass** jeder Vorsprung (510a,510b,510c,610a,610b,610c,810a,810b) vollständig von im Wesentlichen nicht vorspringendem HME-Material umgeben ist.

2. HME-Material (500,600a,600b,600c,800) nach Anspruch 1, wobei die Vorsprünge (510a,510b,510c,610a,610b,610c,810a,810b) nicht die gesamte Breite des HME-Materials (500,600a,600b,600c,800) überspannen.

3. HME-Material (500,600a,600b,600c,800) nach Anspruch 1 oder 2, wobei die Vorsprünge (510a,510b,510c,610a,610b,610c,810a,810b) halbkugelförmig, kegelstumpfförmig, zylindrisch, pyramidenförmig, quaderförmig, halbzylindrisch oder eine beliebige Kombination davon sind.

4. HME-Material (500,600a,600b,600c,800) nach einem vorhergehenden Anspruch, wobei die Dichte der Vorsprünge (510a,510b,510c,610a,610b,610c,810a,810b) pro cm² zwischen 3 und 50, zwischen 5 und 40, zwischen 8 und 30 oder zwischen 10 und 20 ist.

5. HME-Material (500,600a,600b,600c,800) nach einem vorhergehenden Anspruch, wobei jeder Vorsprung (510b,510c) eine Perforation (520b,520c) in dem HME-Material umfasst.

6. HME-Material (500,600a,600b,600c,800) nach Anspruch 5, wobei die Perforationen (520c) Schnitte in dem HME-Material sind, die einen Bereich des HME-Materials teilweise umgeben, sodass dieser Bereich des HME-Materials eine Klappe (530c) bildet, die sich von der Oberfläche des HME-Materials erstreckt.

7. HME-Material (500,600a,600b,600c,800) nach einem vorhergehenden Anspruch, wobei das HME-Material (500,600a,600b,600c,800) Cellulosepapier umfasst.

8. HME-Material (500,600a,600b,600c,800) nach einem vorhergehenden Anspruch, wobei sich einzelne Vorsprünge (510a,510b,510c,610a,610b,610c,810a,810b) von zumindest einem Teil von zwei gegenüberliegenden Oberflächen des HME-Materials erstrecken.

9. HME-Medium (700), umfassend eine Vielzahl von übereinanderliegenden Schichten eines HME-Materials (500,600a,600b,600c,800) nach einem vorhergehenden Anspruch.

10. HME-Medium (700) nach Anspruch 9, wobei das HME-Medium in der Form einer Spule aus dem HME-Material (500,600a,600b,600c,800) ist.

11. HME-Vorrichtung, umfassend ein HME-Medium (700) nach Anspruch 9 oder 10.

12. Verfahren zum Prägen eines HME-Materials (500,600a,600b,600c,800) zur Verwendung bei Wärme- und Feuchtigkeitsaustausch mit Atemgasen, umfassend Drücken eines HME-Materials gegen eine Prägeoberfläche (310a,310b,310c,310d), die eine Vielzahl von einzelnen Überständen (350a,350b,350c,350d,750a) umfasst, **dadurch gekennzeichnet, dass** jeder einzelne Überstand (350a,350b,350c,350d,750a) der Prägeoberfläche (310a,310b,310c,310d) vollständig von im Wesentlichen nicht überstehenden Bereichen der Prägeoberfläche umgeben ist.

13. Verfahren nach Anspruch 12, wobei das HME-Material gegen die Überstände (350a,350b,350c,350d,750a) auf der Prägeoberfläche (310a,310b,310c,310d) durch eine zweite Prägeoberfläche (210a,210b) gedrückt wird, die Aussparungen (250a,250b,750b) umfasst, die den Überständen (350a,350b,350c,350d,750a) der ersten Prägeoberfläche (310a,310b,310c,310d) entsprechen.

14. Verfahren nach Anspruch 13, wobei die zweite Prägeoberfläche ferner Überstände (750a) umfasst, die den Aussparungen (750b) entsprechen, die in der ersten Prägeoberfläche bereitgestellt sind.

15. Verfahren nach einem der Ansprüche 12 bis 14, wobei die Überstände (350a,350b,350c,350d,750a) Perforationen in dem HME-Material (500,600a,600b,600c,800) bilden.

## Revendications

1. Matériau HME (500, 600a, 600b, 600c, 800) destiné à être utilisé dans un échange de chaleur et d'humidité
avec des gaz respiratoires comprenant une pluralité de
de parties saillantes distinctes (510a, 510b, 510c, 610a, 610b, 610c, 810a, 810b) s'étendant à partir d'au moins une partie d'une surface du matériau HME (500, 600a, 600b, 600c, 800), **caractérisé en ce que** chaque partie saillante (510a, 510b, 510c, 610a, 610b, 610c, 810a, 810b) est complètement entouré d'un matériau HME sensiblement non saillant.

2. Matériau HME (500, 600a, 600b, 600c, 800) selon la revendication 1, lesdites parties saillantes (510a, 510b, 510c, 610a, 610b, 610c, 810a, 810b) ne s'étendant pas sur toute la largeur du matériau HME (500, 600a, 600b, 600c, 800).

3. Matériau HME (500, 600a, 600b, 600c, 800) selon la revendication 1 ou 2, lesdites parties saillantes (510a, 510b, 510c, 610a, 610b, 610c, 810a, 810b) étant hémisphériques, tronconiques, cylindriques, pyramidales, cuboïdes, hémicylindriques ou toute combinaison de ceux-ci.

4. Matériau HME (500, 600a, 600b, 600c, 800) selon une quelconque revendication précédente, ladite densité des parties saillantes (510a, 510b, 510c, 610a, 610b, 610c, 810a, 810b) par cm² étant comprise entre 3 et 50, entre 5 et 40, entre 8 et 30 ou entre 10 et 20.

5. Matériau HME (500, 600a, 600b, 600c, 800) selon une quelconque revendication précédente, chaque partie saillante (510b, 510c) comprenant une perforation (520b, 520c) dans le matériau HME.

6. Matériau HME (500, 600a, 600b, 600c, 800) selon la revendication 5,
lesdites perforations (520c) étant des découpes dans le matériau HME qui entourent partiellement une zone de matériau HME, de sorte que cette région de matériau HME forme un rabat (530c) s'étendant à partir de la surface du matériau HME.

7. Matériau HME (500, 600a, 600b, 600c, 800) selon une quelconque revendication précédente, ledit matériau HME (500, 600a, 600b, 600c, 800) comprenant du papier cellulosique.

8. Matériau HME (500, 600a, 600b, 600c, 800) selon une quelconque revendication précédente, des parties saillantes distinctes (510a, 510b, 510c, 610a, 610b, 610c, 810a, 810b) s'étendant à partir d'au moins une partie de deux surfaces opposées du matériau HME.

9. Milieu HME (700) comprenant une pluralité de couches superposées d'un matériau HME (500, 600a, 600b, 600c, 800) selon une quelconque revendication précédente.

10. Milieu HME (700) de la revendication 9, ledit milieu HME se présentant sous la forme d'une bobine du matériau HME (500, 600a, 600b, 600c, 800).

11. Dispositif HME comprenant un milieu HME (700) selon la revendication 9 ou 10.

12. Procédé de gaufrage d'un matériau HME (500, 600a, 600b, 600c, 800) destiné à être utilisé dans un échange de chaleur et d'humidité avec des gaz respiratoires comprenant
la pression d'un matériau HME contre une surface de gaufrage (310a, 310b, 310c, 310d) comprenant une pluralité de parties saillantes distinctes (350a, 350b, 350c, 350d, 750a), **caractérisé en ce que** chaque partie saillante distincte (350a, 350b, 350c, 350d, 750a) de la surface de gaufrage (310a, 310b, 310c, 310d) est complètement entourée par des zones sensiblement non saillantes de la surface de gaufrage.

13. Procédé de la revendication 12, ledit matériau HME étant pressé contre les parties saillantes (350a, 350b, 350c, 350d, 750a) sur la surface de gaufrage (310a, 310b, 310c, 310d) par une seconde surface de gaufrage (210a, 210b) comprenant des évidements (250a, 250b, 750b) qui correspondent aux parties saillantes (350a, 350b, 350c, 350d, 750a) de la première surface de gaufrage (310a, 310b, 310c, 310d).

14. Procédé de la revendication 13, ladite seconde surface de gaufrage comprenant en outre des parties saillantes (750a) qui correspondent à des évidements (750b) prévus dans la première surface de gaufrage.

15. Procédé selon l'une quelconque des revendications 12 à 14, lesdites parties saillantes (350a, 350b, 350c, 350d, 750a) formant des perforations dans le matériau HME (500, 600a, 600b, 600c, 800).
